# EUROPEAN PATENT APPLICATION

(11) **EP 3 505 127 A1**
(43) Date of publication of application: **03.07.2019**
(21) Application number: 17843982.4
(22) Date of filing: 24.08.2017
(51) Int. Cl.: A61B 18/18, A61B 18/12, A61M 25/09, A61M 25/00, A61B 18/00

(54) **DENERVATION CATHETER**

(30) Priority: 25.08.2016 KR 20160108293; 14.08.2017 KR 20170103192
(71) Applicant: Handok Kalos Medical Inc., Seoul 06235 (KR)
(72) Inventor: PARK, Euljoon, Seoul 06102 (KR); OH, Jungsoo, Yongin-si Gyeonggi-do 16999 (KR); PARK, Jae Hyung, Seoul 08844 (KR); LEE, Namha, Seoul 08579 (KR); JANG, Hyunhwan, Gunpo-si Gyeonggi-do 15823 (KR); SONG, Seungwoo, Seoul 05848 (KR); BAE, In Hee, Seoul 06236 (KR); CHO, Jiyong, Seoul 08550 (KR)
(74) Representative: Ström & Gulliksson AB
(86) International application number: PCT/KR2017/009283
(87) International publication number: WO 2018/038562

(57) **Abstract**

Disclosed is a catheter which has a simplified structure and is easy to be downsized. The catheter elongates in one direction and has a proximal end and a distal end, and includes a first support member located near the distal end of the catheter and having a first hollow with both open ends; a second support member located near the proximal end of the catheter in comparison to the first support member and having a second hollow with both open ends; an operating tube configured to elongate in one direction and having a third hollow with both open longitudinal ends, the operating tube being inserted into the first hollow and the second hollow and fixed to the first support member or the second support member to move the first support member or the second support member in a longitudinal direction; at least one connection member having one end connected to the first support member and the other end connected to the second support member, wherein when a distance between the first support member and the second support member decreases, at least a part of the connection member is bent so that the bending portion becomes away from a central axis of the third hollow; at least one electrode provided at the connection member to generate heat; and a shaft body located near the proximal end of the catheter in comparison to the second support member to elongate in one direction, the shaft body having a fourth hollow with both open longitudinal ends so that the operating tube is inserted therein and movable in the longitudinal direction.

## Description

### TECHNICAL FIELD

The present application claims priority to Korean Patent Application No. 10-2016-0108293 filed on August 25, 2016 and No. 10-2017-0103192 filed on August 14, 2017 in the Republic of Korea, the disclosures of which are incorporated herein by reference.

The present disclosure relates to a catheter, particularly to a medical catheter for treating diseases, more particularly to a catheter for denervation, which ablates a part of nerves to inactivate nerve conduction, and a denervation apparatus having the catheter.

### BACKGROUND ART

Denervation is a surgical procedure for blocking a part of nerve paths for various nerves such as sensory nerves and automatic nerves so that stimulation or information is not delivered. The denervation is being used more and more for treatment of several diseases such as arrhythmia, pain relief, plastic surgery or the like.

In particular, as it has been recently reported that the denervation is available for treatment of hypertension, many endeavors are being made to apply the denervation for effective treatment of hypertension.

In case of hypertension, since blood pressure can be mostly controlled with drugs, most hypertensive patients are depending on drugs until now. However, if blood pressure is lowered with drugs, a hypertensive patient should take the drugs continually, which causes inconvenience and increases costs. In addition, if drugs are taken for a long time, various problems such as damage to internal organs or other side effects. Moreover, some hypertensive patients suffer from intractable hypertension which does not allow easy control of blood pressure with drugs. Since the intractable hypertension is not treated with drugs, the possibility of accidents such as a stroke, an irregular heartbeat, a kidney disease or the like increases. Therefore, the treatment of intractable hypertension is a very serious and urgent issue.

In this circumstance, the denervation attracts attention as an innovative scheme to treat hypertension. In particular, the denervation for treating hypertension may be performed by ablating sympathetic nerves around renal nerves, namely the renal artery, to inactivate nerve conduction so that the renal nerves are blocked. If the renal nerve is activated, the production of renin hormone increases by the kidney, which may cause the increase of blood pressure. Therefore, if the renal nerve is blocked, nerve conduction is not performed, and thus the hypertension may be treated, as proven by various recent experiments.

As described above, a representative renal denervation for treating hypertension is using a catheter. In the denervation using a catheter, a catheter is inserted into a part of a human body, for example the thigh, and a distal end of the catheter is located at the renal artery. In this state, heat is generated at the distal end of the catheter by means of radio frequency (RF) energy or the like to block sympathetic nerves around the renal artery.

If the denervation using a catheter is performed, a very small region is cut in a human body in comparison to the denervation using an abdominal operation. Therefore, latent complications or side effects may greatly decrease, and the time taken for treatment or recovery is very short due to local anesthesia. Therefore, the denervation using a catheter is spotlighted as a next-generation hypertension treatment method due to the above advantages.

However, the catheter-related technology to be applied to the denervation or the like is insufficiently developed until now and thus has many parts to be improved.

In particular, the catheter should be small enough to freely move along the interior of a blood vessel. However, in the existing technique, there are many difficulties in downsizing the catheter.

Moreover, the head of the catheter which has been developed or proposed is provided with at least one electrode and various sensing devices and also includes various wires for transmitting electric power or electrical signals to the electrode and the sensing devices. Therefore, in the existing technique, it is very difficult to manufacture a catheter having all of these components with a small size.

Further, in some catheters, if a distal end of the catheter reaches a surgical operation site, the head is inflated so that the electrode approaches an inner wall of the blood vessel. At this time, in order to allow an operator to expand or shrink the catheter head, a separate wire may be provide to elongate from a distal end to a proximal end of the catheter. In this case, the internal structure of the catheter may become more complicated and larger due to the wire, which may add difficulties in downsizing the catheter. In addition, in many cases, the wire is made of a metal material, and the wire made of a metal may be exposed to the outside at the head of the catheter and come into contact with blood, thus causing various problems.

### DISCLOSURE

### Technical Problem

The present disclosure is designed to solve the problems of the related art, and therefore the present disclosure is directed to providing a catheter which has a simplified structure and is easy to be downsized.

These and other objects and advantages of the present disclosure may be understood from the following detailed description and will become more fully apparent from the exemplary embodiments of the present disclosure. Also, it will be easily understood that the objects and advantages of the present disclosure may be realized by the means shown in the appended claims and combinations thereof.

### Technical Solution

In one aspect of the present disclosure, there is provided a catheter, which elongates in one direction and has a proximal end and a distal end, the catheter comprising: a first support member located near the distal end of the catheter and having a first hollow with both open ends; a second support member located near the proximal end of the catheter in comparison to the first support member and having a second hollow with both open ends; an operating tube configured to elongate in one direction and having a third hollow with both open longitudinal ends, the operating tube being inserted into the first hollow and the second hollow and fixed to the first support member or the second support member to move the first support member or the second support member in a longitudinal direction; at least one connection member having one end connected to the first support member and the other end connected to the second support member, wherein when a distance between the first support member and the second support member decreases, at least a part of the connection member is bent so that the bending portion becomes away from a central axis of the third hollow; at least one electrode provided at the connection member to generate heat; and a shaft body located near the proximal end of the catheter in comparison to the second support member to elongate in one direction, the shaft body having a fourth hollow with both open longitudinal ends so that the operating tube is inserted therein and movable in the longitudinal direction.

Here, the catheter according to the present disclosure may further comprise a guide wire configured to elongate in one direction and inserted into the third hollow through both open ends of the operating tube to be movable in the longitudinal direction inside the third hollow.

In addition, the operating tube may be fixed to the first support member and be movable in the longitudinal direction inside the second hollow.

In addition, the operating tube may include: an inner tube configured to elongate in one direction and having the third hollow; a mesh tube having a mesh form and configured to surround an outer surface of the inner tube; and an outer tube configured to elongate in one direction and surround the outer surfaces of the inner tube and the mesh tube.

In addition, the operating tube may include: an outer tube configured to elongate in one direction and having the third hollow; and a coil tube having a coil form and configured to surround an inner surface of the outer tube.

In addition, the shaft body may include: an inner body configured to elongate in one direction and having the fourth hollow; a mesh body having a mesh form and configured to surround an outer surface of the inner body; and an outer body configured to elongate in one direction and surround the outer surfaces of the inner body and the mesh body.

In addition, the catheter according to the present disclosure may further comprise a power supply wire electrically connected to the electrode to give a power supply path to the electrode.

In addition, the shaft body may further have a fifth hollow formed therein in the longitudinal direction, separately from the fourth hollow, and the power supply wire may be configured to be inserted into the fifth hollow and movable in the longitudinal direction, and be connected from the proximal end of the catheter to the electrode.

In addition, the fifth hollow may be provided at one outer side of the fourth hollow and is bent along an outer circumference of the fourth hollow.

In addition, the shaft body may be configured so that a central axis of the fourth hollow coincides with a central axis of the catheter, and the power supply wire may be provided in plural so that the power supply wires are arranged to be spaced apart from each other by a predetermined angle on the basis of the central axis of the fourth hollow.

In addition, the power supply wires may be inserted between an inner surface and an outer surface of the shaft body and be fixedly coupled to the shaft body.

In addition, the power supply wires may be inserted between an inner surface and an outer surface of the operating tube and be fixedly coupled to the operating tube.

In addition, the power supply wire may be provided in contact with an outer surface of the operating tube, and the catheter may further comprise a thermally shrinkable film having a thermally shrunken form to surround outer sides of the power supply wire and the operating tube.

In addition, the catheter according to the present disclosure may further comprise a deflection wire configured to elongate in one direction and having a distal end fixed to a distal end of the shaft body and a proximal end exposed outwards at a proximal end of the shaft body, the deflection wire being configured to be movable in the longitudinal direction inside the shaft body.

In addition, in another aspect of the present disclosure, there is also provided a denervation apparatus, which comprises the catheter according to the present disclosure.

### Advantageous Effects

According to the present disclosure, a medical catheter, especially a catheter for effective denervation, is provided.

According to an embodiment of the present disclosure, a head of the catheter may be opened or closed by using a tube member which allows a guide wire to pass therethrough.

Therefore, it is not necessary to separately provide a wire-shaped operating member for operating the catheter head in the catheter.

For this reason, the catheter may have a simplified structure and a reduced volume. Further, since the operating member made of a metal is not exposed to the blood, various problems caused by the operating member may be prevented.

In addition, according to an embodiment of the present disclosure, the catheter head may be made flexible because it is not necessary to provide a wire-shaped operating member made of a metal material.

### DESCRIPTION OF DRAWINGS

The accompanying drawings illustrate a preferred embodiment of the present disclosure and together with the foregoing disclosure, serve to provide further understanding of the technical features of the present disclosure, and thus, the present disclosure is not construed as being limited to the drawing.
FIG. 1 is a perspective view schematically showing a configuration of a catheter according to an embodiment of the present disclosure at a distal end thereof.
FIG. 2 is a perspective view schematically showing a first support member according to an embodiment of the present disclosure.
FIG. 3 is a perspective view schematically showing an operating tube of the catheter according to an embodiment of the present disclosure at a distal end thereof.
FIGS. 4 and 5 are front views schematically showing that the first support member is moved due to the movement of the operating tube according to an embodiment of the present disclosure.
FIG. 6 is a perspective view schematically showing an interior configuration of the operating tube according to an embodiment of the present disclosure.
FIG. 7 is a perspective view schematically showing a configuration of an operating tube according to another embodiment of the present disclosure.
FIG. 8 is a perspective view schematically showing an operating tube according to another embodiment of the present disclosure.
FIG. 9 is a perspective view schematically showing a shaft body having a hollow in which the operating tube according to an embodiment of the present disclosure is inserted.
FIG. 10 is a cross-sectioned view, taken along the line BI-Bl' of FIG. 9.
FIG. 11 is a perspective view schematically showing a catheter according to another embodiment of the present disclosure.
FIG. 12 is a cross-sectioned view, taken along the line B2-B2' FIG. 11.
FIGS. 13 and 14 are schematic views showing that a power supply wire is included in the shaft body according to an embodiment of the present disclosure.
FIG. 15 is a perspective view schematically showing a catheter according to another embodiment of the present disclosure.
FIG. 16 is a cross-sectioned view, taken along the line B3-B3' of FIG. 15.
FIGS. 17 and 18 are schematic views showing that a power supply wire is included in the operating tube according to an embodiment of the present disclosure.
FIG. 19 is a schematic view showing that a power supply wire is provided at an outer side of the operating tube according to another embodiment of the present disclosure.
FIG. 20 is a schematic view showing a catheter to which the configuration of FIG. 19 is applied.

### BEST MODE

Hereinafter, preferred embodiments of the present disclosure will be described in detail with reference to the accompanying drawings. Prior to the description, it should be understood that the terms used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation.

Therefore, the description proposed herein is just a preferable example for the purpose of illustrations only, not intended to limit the scope of the disclosure, so it should be understood that other equivalents and modifications could be made thereto without departing from the scope of the disclosure.

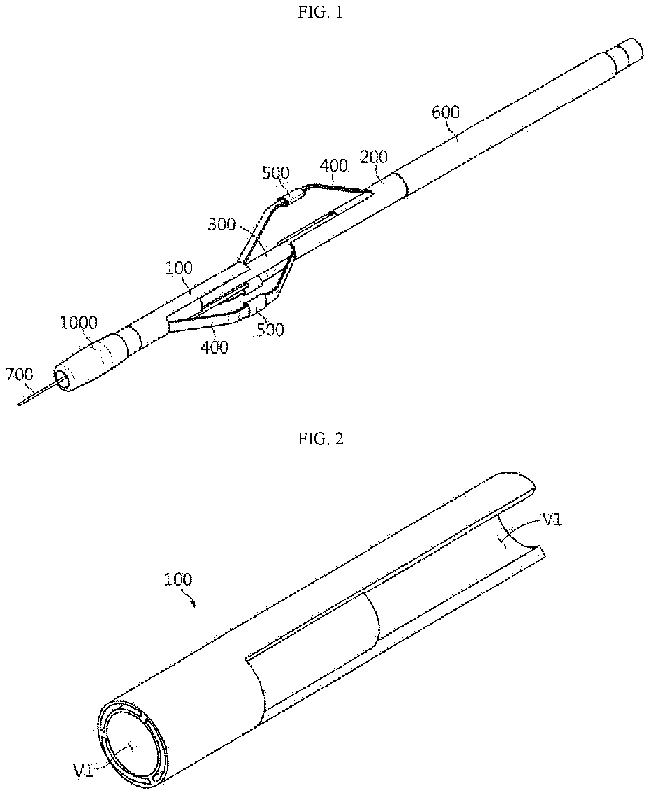

FIG. 1 is a perspective view schematically showing a configuration of a catheter according to an embodiment of the present disclosure at a distal end thereof.

Here, the distal end of the catheter means an end of the catheter which reaches a surgical operation site, between both ends of the catheter in a longitudinal direction. In other words, the catheter elongates in one direction with both ends and may be configured to move along an inner space of a blood vessel or the like. At this time, the end of the catheter located toward a surgical operator may be called a proximal end, and the end of the catheter that is located opposite to the proximal end and reaches a surgical operation site for the first time may be called a distal end. The surgical operator may be located near the proximal end of the catheter and adjust the movement of the distal end of the catheter. The distal end of the catheter may also be called a catheter head. Hereinafter, regarding not only the catheter but also various components of the catheter which extend in the longitudinal direction of the catheter and thus have both ends in the longitudinal direction, an end of a component, located at the distal end of the catheter, will be called a distal end of the corresponding component, and a proximal end of a component, located at the proximal end of the catheter, will be called a proximal end of the corresponding component.

Referring to FIG. 1, the catheter according to the present disclosure may include a first support member 100, a second support member 200, an operating tube 300, a connection member 400, an electrode 500, and a shaft body 600.

The first support member 100 may be located near the distal end of the catheter. In other words, the first support member 100 may be located at the end of the catheter, which is not near a surgical operator but near a surgical operation site. Also, the first support member 100 may have a hollow therein. The configuration of the first support member 100 will be described in more detail with reference to FIG. 2.

FIG. 2 is a perspective view schematically showing the first support member 100 according to an embodiment of the present disclosure.

Referring to FIG. 2, the first support member 100 may be configured in a cylinder form with a hollow therein as indicated by V1. The hollow may be formed along the longitudinal direction of the catheter and have both ends opened. In other words, in the configuration of FIG. 2, the hollow of the first support member 100 may be formed substantially in a right and left direction, so that a left end and a right end thereof are opened, respectively. Therefore, a predetermined component may be inserted into the hollow of the first support member 100. In this specification, the hollow formed in the first support member 100 is called a first hollow, so as to be distinguished from hollows formed in other components.

The second support member 200 may be located near the proximal end of the catheter in comparison to the first support member 100. In other words, the second support member 200 may be located near the distal end based on the entire portion of the catheter, but near the proximal end of the catheter in comparison to the first support member 100. For example, as shown in FIG. 1, the second support member 200 may be configured to be spaced from the first support member 100 by a predetermined distance and be located near a right side of the catheter, which may be referred to as the proximal end of the catheter, in comparison to the first support member 100.

In addition, the second support member 200 may have a hollow therein, similar to the first support member 100. The hollow of the second support member 200 may be formed along the longitudinal direction of the catheter and have both ends opened. For example, the second support member 200 may be configured symmetrically in the longitudinal direction of the catheter with respect to the first support member 100 shown in FIG. 2. Thus, certain components may be inserted into the hollow of the second support member 200. In particular, the operating tube 300 may be inserted into the hollow of the second support member 200.

In this specification, the hollow formed in the second support member 200 is called as a second hollow, so as to be distinguished from hollows formed in other components. The second hollow may also have both ends opened.

The first support member 100 and the second support member 200 may be spaced apart from each other along the longitudinal direction of the catheter. In addition, the distance between the first support member 100 and the second support member 200 may be changed. The configuration for changing the distance between them will be described later.

The first support member 100 and/or the second support member 200 may be made of various materials having biocompatibility. For example, the support members may be made of not only a flexible material such as rubber or plastic but also a hard material such as metal.

In particular, the support members may be made of a soft and flexible material. Further, the first support member 100 is located at a front end of the catheter and is highly likely to contact the inner wall of a blood vessel when the catheter moves along the blood vessel. Thus, the first support member 100 is made of a soft and flexible material to prevent the blood vessel from being damaged and to allow easy change of direction.

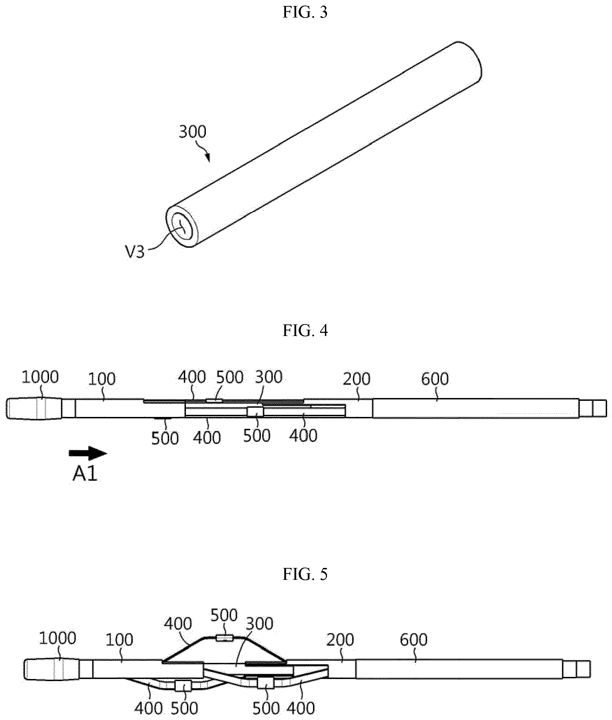

The operating tube 300 may be configured in a tube form, namely a tubular shape. The configuration of the operating tube 300 is shown will be described in more detail with reference to FIG. 3.

FIG. 3 is a perspective view schematically showing the operating tube 300 of the catheter according to an embodiment of the present disclosure at a distal end thereof.

Referring to FIG. 3, the operating tube 300 may be configured to elongate in one direction. Here, the longitudinal direction of the operating tube 300 may be regarded as being consistent with the longitudinal direction of the catheter. In particular, the operating tube 300 may extend from the distal end of the catheter to the proximal end of the catheter. Thus, the distal end of the operating tube 300 is located near a surgical operation site, and the proximal end of the operating tube 300 may be located near a surgical operator. Thus, the surgical operator may move the operating tube 300 in the longitudinal direction by manipulating the proximal end of the operating tube 300.

The operating tube 300 may have a hollow formed along the longitudinal direction thereof, as indicated by V3. In addition, both ends of the hollow of the operating tube 300 may be opened. For example, as shown in FIG. 3, the operating tube 300 may be formed to elongate substantially in a right and left direction so that both a left end and a right end of the hollow are opened. In this specification, the hollow of the operating tube 300 is called a third hollow, so as to be distinguished from hollows of the other components.

As described above, the operating tube 300 has a hollow therein, and both ends of the hollow are opened, so that any component may be inserted into the operating tube 300 or move inside the operating tube 300.

The operating tube 300 may be inserted into both the first hollow V1, which is the hollow of the first support member 100, and the second hollow, which is the hollow of the second support member 200. In other words, the operating tube 300 may be configured such that a portion of the outer surface thereof is surrounded by the first support member 100 and the second support member 200. At this time, since the first support member 100 and the second support member 200 are located near the distal end of the catheter, it may be regarded that a portion of the distal end of the operating tube 300 is surrounded by the first support member 100 and the second support member 200.

The operating tube 300 may be fixed to the first support member 100 or the second support member 200. In other words, the operating tube 300 may be fixed to the first support member 100 or the second support member 200 in a state of being inserted into the hollows of the first support member 100 and the second support member 200, respectively. In addition, since the operating tube 300 is fixed to the first support member 100 or the second support member 200 as described above, the first support member 100 or the second support member 200 may be moved in the longitudinal direction when the operating tube 300 moves in the longitudinal direction. This movement configuration will be described in more detail with reference to FIGS. 4 and 5.

FIGS. 4 and 5 are front views schematically showing that the first support member 100 is moved due to the movement of the operating tube 300 according to an embodiment of the present disclosure.

First, referring to FIG. 4, the first support member 100 and the second support member 200 are spaced apart from each other by a predetermined distance, and the operating tube 300 is inserted into the hollows of the first support member 100 and the second support member 200. At this time, the operating tube 300 may be configured to move freely in the longitudinal direction, namely in a right and left direction on the figure, in a state of being inserted into the hollow of the second support member 200. For this, an outer diameter of the operating tube 300 may be slightly smaller than an inner diameter of the second support member 200. In addition, the operating tube 300 is fixedly coupled to the first support member 100 in a state of being inserted into the hollow of the first support member 100.

In this configuration, if the operating tube 300 is moved in a direction A1, the first support member 100 fixedly coupled to the operating tube 300 may move in a right direction as shown in FIG. 5. In other words, if a surgical operator pulls the proximal end of the operating tube 300, the operating tube 300 moves in the direction A1. At this time, since the operating tube 300 may freely move in the longitudinal direction inside the hollow of the second support member 200, the position of the second support member 200 may not change. However, since the first support member 100 is fixed to the operating tube 300, when the operating tube 300 moves in the direction A1, the first support member 100 may also move in the direction A1. On the contrary, if the surgical operator pushes the proximal end of the operating tube 300, the operating tube 300 moves in a direction opposite to the direction A1, which may cause the first support member 100 to move in the direction opposite to the direction A1. Then, the configuration of the distal end of the catheter may be changed from the configuration of FIG. 5 into the configuration of FIG. 4.

As described above, if the operating tube 300 moves the first support member 100 or the second support member 200 in the longitudinal direction, the distance between the first support member 100 and the second support member 200 may be changed. For example, if the operating tube 300 moves in the direction A1 in the configuration of FIG. 4, the distance between the first support member 100 and the second support member 200 may be decreased as in the configuration of FIG. 5. On the contrary, if the operating tube 300 moves in the direction opposite to the direction A1 in the configuration of FIG. 4, the distance between the first support member 100 and the second support member 200 may be increased.

The connection member 400 may be provided between the first support member 100 and the second support member 200 to connect the first support member 100 and the second support member 200 to each other. In other words, one end of the connection member 400 may be fixed to the first support member 100, and the other end of the connection member 400 may be fixed to the second support member 200. The connection member 400 may have a bar shape or a plate shape elongating in one direction.

The connection member 400 may be configured such that at least a portion of the connection member 400 is bent when the distance between the first support member 100 and the second support member 200 is decreased, since the distance of both ends thereof is decreased. In addition, this bending portion of the connection member 400 may be configured to become away from the central axis of the catheter. In other words, if the first support member 100 and the second support member 200 become closer to each other so that the connection member 400 is bent, the bending portion may become away from the central axis of the operating tube 300.

In particular, in the catheter according to the present disclosure, the distance between the first support member 100 and the second support member 200 may be adjusted by the operating tube 300. In other words, as the operating tube 300 moves in the longitudinal direction, the distance between the first support member 100 and the second support member 200 may be changed. Accordingly, in the catheter according to the present disclosure, the connection member 400 may be bent or spread by means of the movement of the operating tube 300.

Meanwhile, the connection member 400 may be made of a material which is bendable when the distance between both ends thereof is narrowed, because a bending portion should be formed according to the movement of the first support member 100 or the second support member 200. For example, the connection member 400 may be made of a material such as a metal or a polymer. However, the connection member 400 of the present disclosure is not limited to any specific material, and the connection member 400 may be made of various materials as long as a bending portion may be formed in a portion of the connection member 400.

A plurality of connection members 400 may be provided between the first support member 100 and the second support member 200. For example, three connection members 400 may be provided as shown in FIG. 1. However, the present disclosure is not limited to the specific number of connection members 400, and the connection member 400 may be configured in various numbers. If a plurality of connection members 400 are provided as described above, each connection member 400 is bent at least partially when the distance between the first support member 100 and the second support member 200 is decreased, so that the bending portion becomes away from the central axis of the operating tube 300.

The electrode 500 is mounted to the connection member 400 and may generate heat with a supplied power. In addition, the heat generated by the electrode 500 in this way may apply a thermal stimulation to surrounding tissues. For example, the heat generated by the electrode 500 may ablate the surrounding tissues. At this time, the electrode 500 may generate heat at about 40 to 80°C to ablate the nerve around a blood vessel, which may block the nerve. However, the temperature of the heat generated by the electrode 500 may be implemented in various ways according to the use or purpose of the catheter.

The electrode 500 may apply heat to the nerve tissues located around a blood vessel in contact with a wall of the blood vessel, and thus the electrode 500 may be designed to closely adhere to the wall of the blood vessel. Therefore, the electrode 500 may be formed in a curved shape such that the surface of the electrode 500 in contact with the inner wall of the blood vessel may correspond to the shape of the inner wall. For example, the electrode 500 may be configured to have a circular, semi-circular, or elliptical section.

In particular, the electrode 500 may be provided at the bending portion of the connection member 400. The bending portion of the connection member 400 may be configured to be farthest away from the central axis of the catheter, as the distance between the first support member 100 and the second support member 200 is changed. Thus, if the electrode 500 is provided at the bending portion of the connection member 400, the electrode 500 may be positioned closest to the inner wall of the blood vessel.

In this configuration, if the distance between the first support member 100 and the second support member 200 is decreased so that the connection member 400 is bent greatest, the electrode 500 may approach the inner wall of the blood vessel, and in this case, the catheter head may be regarded as being opened. On the contrary, if the distance between the first support member 100 and the second support member 200 is increased such that the connection member 400 is spread or bent smallest, the electrode 500 may be farthest from the inner wall of the blood vessel, and in this case, the catheter head may be regarded as being closed.

The electrode 500 may be made of material such as platinum or stainless steel, but the present disclosure is not limited to such specific materials of the electrode 500, and the electrode 500 may be made of various materials in consideration of various factors such as a heat generation type and an operation target.

The electrode 500 may generate heat by means of radio frequency (RF). For example, the electrode 500 may be electrically connected to a high frequency generating unit to emit high frequency energy and thus ablate nerves.

Meanwhile, the electrode 500 provided at the catheter may be a negative electrode, and a positive electrode corresponding to the negative electrode may be connected to an energy supplying unit such as a high frequency generating unit, similar to the negative electrode, and attached to a specific portion of a human body in the form or patch or the like.

The catheter may include two or more electrodes 500. In particular, if two or more connection members 400 are included in the catheter, the electrode 500 may be provided for each connection member 400. For example, as shown in FIG. 1, the catheter according to the present disclosure may include three connection members 400 and three electrodes 500, so that one electrode 500 is mounted to the bending portion of each connection member 400.

The shaft body 600 may be configured to elongate in one direction. For example, as shown in FIG. 1, the shaft body 600 may be configured to elongate substantially in a right and left direction. In particular, the shaft body 600 may be configured to extend from the distal end of the catheter where the first support member 100 and the second support member 200 are located to the proximal end of the catheter where a surgical operator is located.

The shaft body 600 may be located near the proximal end of the catheter in comparison to the second support member 200. For example, as shown in FIG. 1, the shaft body 600 may be located at a right side in comparison to the second support member 200. At this time, the shaft body 600 may be in contact with the second support member 200. In other words, in the configuration of FIG. 1, the left end (the distal end) of the shaft body 600 may be configured to be in contact with the right end (the proximal end) of the second support member 200.

Here, the shaft body 600 and the second support member 200 may be fixed in contact with each other. In this case, the space between the second support member 200 and the shaft body 600 may not be exposed. Thus, it is possible to prevent blood or foreign matter from penetrating into the hollow of the second support member 200 or the outer surface of the operating tube 300.

The shaft body 600 may have a hollow formed in the longitudinal direction. For example, in the configuration shown in FIG. 1, the shaft body 600 may have a hollow formed substantially in the right and left direction in the inner space. In this specification, the hollow formed in the shaft body 600 is called a fourth hollow, so as to be distinguished from hollows formed in other components.

This fourth hollow may have both ends opened in the longitudinal direction. For example, in the case where the shaft body 600 is elongated in the right and left direction, the fourth hollow may be formed long in the right and left direction, and the left end and the right end of the fourth hollow may be opened, respectively. The operating tube 300 may be inserted into this fourth hollow and be movable in the longitudinal direction. In other words, if a surgical operator grips the proximal end of the operating tube 300 and move the proximal end in the longitudinal direction, the operating tube 300 may move freely in the longitudinal direction in a state of being inserted into the fourth hollow. At this time, the outer surface of the operating tube 300 and the surface of the fourth hollow may be spaced at least partially by a predetermined distance so that the operating tube 300 may freely move inside the fourth hollow. In other words, the diameter of the fourth hollow may be a little larger than the outer diameter of the operating tube 300.

The shaft body 600 occupies most of the length of the catheter and may be located outside the catheter. Also, the shaft body 600 may be mostly located inside a blood vessel. Accordingly, the shaft body 600 may be made of a flexible and bendable material that is biocompatible, easy to move inside the blood vessel and flexed according to the shape of the blood vessel.

The catheter according to the present disclosure may further include a guide wire 700.

The guide wire 700 is a wire to guide the catheter to a surgical operation site and may be configured to reach the surgical operation site prior to the catheter. The guide wire 700 may be configured to elongate in one direction. In addition, the guide wire 700 may be inserted into the hollow of the operating tube 300. In other words, the guide wire 700 may be configured to be inserted into the third hollow V3 through both open ends of the operating tube 300 and to be movable in the longitudinal direction inside the third hollow. In particular, the guide wire 700 may have a wire form and be configured with a significantly smaller diameter than the operating tube 300 and the like. Thus, the guide wire 700 may be freely moved in the longitudinal direction inside the hollow of the operating tube 300. In this configuration, the guide wire 700 moves along the inside of the blood vessel and reaches the surgical operation site first, and the operating tube 300, the first support member 100, the second support member 200, the connection member 400 and the like may be moved to the surgical operation site in a state where the guide wire 700 is inserted into the hollow.

In this case, the operating tube 300 may serve as a component for allowing the guide wire 700 to pass therethrough. In addition, as described above, the operating tube 300 may be used to open or close the catheter head. Thus, in this configuration of the present disclosure, a component that allows the guide wire 700 to pass may serve as a component that regulates opening/closing of the catheter head. Therefore, there is no need for a separate operating member to open/close the catheter head, besides the operating tube 300 for moving the catheter through the guide wire 700.

In particular, in the case of some catheters in the existing technique, a separate operating member having a wire form is required in addition to the component for allowing the guide wire 700 to pass in order to open/close the catheter head. However, in this case, the operating member should be long from the distal end to the proximal end of the catheter, and thus the catheter inevitably has a large diameter in most regions. Meanwhile, according to the embodiment of the present disclosure, the catheter may have a reduced diameter because the component for allowing the guide wire 700 to pass may open/close the catheter head. For example, the shaft body 600 just needs a space in which the operating tube 300 may be moved, and there is no need to separately prepare a space for moving the separate operating member. Thus, the shaft body 600 may have a reduced diameter.

In addition, according to an embodiment of the present disclosure, it is not needed to use a metallic operating member for opening/closing the catheter head or exposing the same to the outside, and thus any problem caused by corrosion or the like may not occur. Moreover, since the metallic operating member of the existing technique is not provided, the catheter, particularly the head of the catheter, may be more flexible.

Meanwhile, as explained in the former embodiment of FIGS. 4 and 5, the operating tube 300 may be configured to be fixed to the first support member 100 and movable in the longitudinal direction inside the second hollow.

According to this configuration of the present disclosure, the operating tube 300 may be configured not to protrude more distally than the first support member 100, regardless of the opened or closed state of the catheter head. For example, in the configuration of FIG. 4, in order to open the catheter head, the operating tube 300 may move in the direction A1, and in order to close the catheter head, the operating tube 300 may move in a direction opposite to the direction A1. At this time, since the distal end of the operating tube 300 is fixed to the first support member 100, the first support member 100 moves together with the movement of the operating tube 300, and the distal end of the operating tube 300 may be configured not to protrude to the left side of the first support member 100. Thus, it is possible to prevent the protruding end of the operating tube 300 from damaging the blood vessel, while the operating tube 300 is being manipulated to open or close the catheter head.

In addition, in this configuration of the present disclosure, there is no risk that the operating tube 300 deviates from the first hollow. For example, in the embodiment of FIG. 4, if the operating tube 300 moves in the right direction, since the operating tube 300 is fixed to the first support member 100, there is no risk that the distal end of the operating tube 300 moves out of the hollow of the first support member 100 to separate the first support member 100 and the operating tube 300 from each other.

Moreover, in this configuration of the present disclosure, the coupling state of the second support member 200 and the shaft body 600 may be stably maintained. For example, in the embodiment of FIG. 4, since the operating tube 300 may be freely moved in a state of being inserted into the hollow of the second support member 200, the position of the second support member 200 may not be changed even if the operating tube 300 is moved in the right and left direction. Thus, the relative positions of the second support member 200 and the shaft body 600 may be maintained stably.

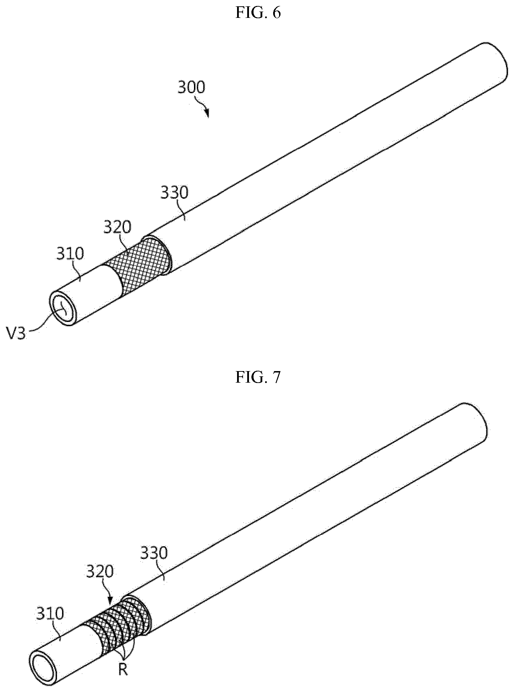

Preferably, the operating tube 300 may be configured to include an inner tube, a mesh tube, and an outer tube 330. This configuration will be described in more detail with reference to FIG. 6.

FIG. 6 is a perspective view schematically showing an interior configuration of the operating tube 300 according to an embodiment of the present disclosure. In FIG. 6, the distal ends of the mesh tube 320 and the outer tube 330 are partially sectioned to show each component therein.

Referring to FIG. 6, the operating tube 300 may have a triple structure having three layers of the inner tube 310, the mesh tube 320 and the outer tube 330.

Here, the inner tube 310 constitutes an inner layer of the operating tube 300, and a hollow may be formed in the longitudinal direction. In other words, the operating tube 300 may elongate in one direction and have a tubular form with a hollow. At this time, since the hollow formed in the inner tube 310 may be regarded as the hollow formed in the operating tube 300, the hollow of the inner tube 310 may be the third hollow. For example, the inner tube 310 may be made of a polymer material.

In addition, the mesh tube 320 may be configured to surround the outer surface of the inner tube 310. Further, the mesh tube 320 may be configured to extend from the distal end to the proximal end of the inner tube 310. In particular, the mesh tube 320 may be configured in a mesh form, or a net form. For example, the mesh tube 320 may be configured in a form having a plurality of wires woven, namely a woven form.

In particular, the mesh tube 320 may be made of a material reinforcing the rigidity of the inner tube 310. For this, the mesh tube 320 may be made of a material with greater rigidity than the inner tube 310. For example, when the inner tube 310 is made of a polymer material, the mesh tube 320 may be made of a metal material. For example, the mesh tube 320 may be made of stainless steel.

The outer tube 330 may be configured to elongate in one direction, similar to the inner tube 310. In particular, the outer tube 330 may be configured to surround the outer surfaces of the inner tube 310 and the mesh tube 320.

The outer tube 330 may be made of a material identical to or different from the inner tube 310, for example a polymer material. For example, the outer tube 330 may be made of PTFE (Polytetrafluoroethylene), Pebax (Poly Ether Block Amides), or the like. In addition, the outer tube 330 may be configured to cover the outer side of the mesh tube 320 so that the mesh tube 320 is not exposed to the outside. In particular, the outer tube 330 may allow the mesh tube 320 to be embedded in the operating tube 300 together with the inner tube 310 without being exposed to the outside.

If this configuration of the present disclosure is applied, the rigidity of the operating tube 300 may be reinforced to facilitate opening/closing of the catheter head by the operating tube 300. In other words, in the present disclosure, as the operating tube 300 moves in the longitudinal direction, the first support member 100 or the second support member 200 moves. For this, the operating tube 300 should receive a certain level of force and should not be bent or pressed easily in the longitudinal direction. Here, according to the above structure, the operating tube 300 may have reinforced rigidity by the triple structure including the mesh structure and receive a certain level of force.

In this configuration, the mesh tube 320 may have a plurality of mesh rings disposed along the longitudinal direction of the inner tube 310 at the outer surface of the inner tube 310. This will be described in more detail with reference to FIG. 7.

FIG. 7 is a perspective view schematically showing a configuration of an operating tube 300 according to another embodiment of the present disclosure. In FIG. 7, the distal ends of the mesh tube 320 and the outer tube 330 are partially sectioned to show each component therein.

Referring to FIG. 7, the operating tube 300 may include an inner tube 310, a mesh tube 320 and an outer tube 330, similar to FIG. 6, but the mesh tube 320 may be configured to include a plurality of unit rings as indicated by R. Here, the plurality of unit rings may have a mesh form in which wires or strings are interwoven, respectively, and may be configured so that at least some parts thereof are separable from each other. For example, the mesh tube 320 is made of stainless steel, and a plurality of such metal ring-shaped members may arranged successively from the distal end to the proximal end of the operating tube 300 to surround the outer surface of the inner tube 310.

According to this configuration of the present disclosure, the mesh tube 320 is configured to have a plurality of unit rings which may be separated from each other, so that the operating tube 300 may be easily bent as needed. In other words, since the catheter moves along the blood vessel, the catheter needs to bend depending on the shape of the blood vessel. Thus, in this embodiment including a plurality of mesh rings, it is possible that the operating tube 300 easily bends by varying the distance between the meshing rings, while easily receiving the force in the longitudinal direction.

Meanwhile, in the operating tube 300 in which the mesh tube 320 is interposed between the inner tube 310 and the outer tube 330, the distal ends of the inner tube 310 and the outer tube 330 may be fused to each other. For example, if the mesh tube 320 is made of a metal material and both the inner tube 310 and the outer tube 330 are made of a polymer material, the distal end of the inner tube 310 and the distal end of the outer tube 330 may be melted and fused to each other by means of thermal treatment.

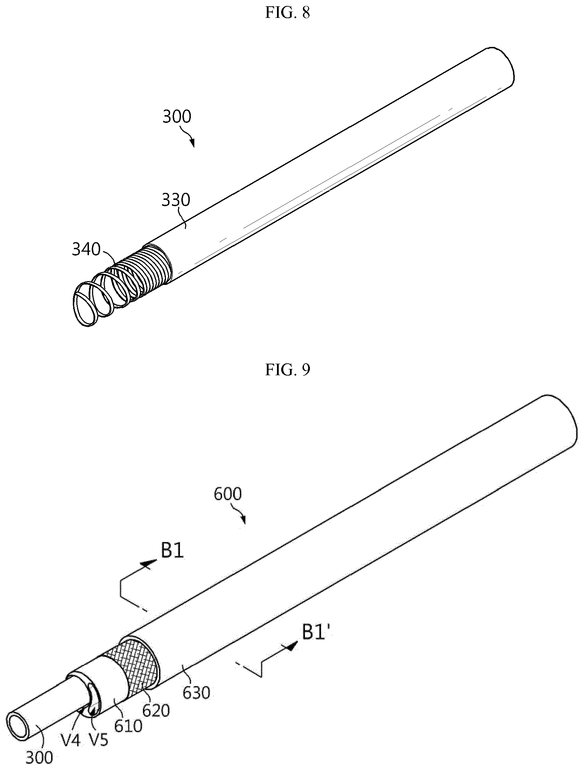

Meanwhile, even though it has been described in the embodiment of FIGS. 6 and 7 that the mesh structure is provided in the operating tube, the present disclosure is not limited to this embodiment. This will be described in more detail with reference to FIG. 8.

FIG. 8 is a perspective view schematically showing an operating tube 300 according to another embodiment of the present disclosure. In FIG. 8, distal ends of some components are partially sectioned to show each component therein.

Referring to FIG. 8, the operating tube 300 according to another embodiment of the present disclosure may include an outer tube 330 and a coil tube 340.

The outer tube 330 may be configured with a pipe form elongating in one direction and having the third hollow extending in the longitudinal direction of the catheter. Moreover, the features of the outer tube described in the former embodiment may be applied identically or similarly to the outer tube 330.

The coil tube 340 may be configured to surround the inner surface of the outer tube 330. In particular, the coil tube 340 may be configured in a spiral form, namely a coil form, wound along the inner surface of the outer tube 330. In FIG. 8, it is depicted that the distal end of the coil tube 340 is pulled distally to show more clearly that the coil tube 340 has a coil form. Moreover, the coil tube 340 may be configured to be wound long from the distal end to the proximal end of the outer tube 330. For example, the coil tube 340 may have a wire form whose section perpendicular to the longitudinal direction has a circular or elliptical shape, such that one end is located at the distal end of the outer tube 330 and the other end is located at the proximal end of the outer tube 330. Alternatively, the coil tube 340 may be configured in a plate form whose thickness is smaller than the width and whose width is remarkably smaller than the length, similar to a wire form.

The coil tube 340 may be made of a material that reinforces the rigidity of the operating tube 300, particularly the outer tube 330. In other words, the coil tube 340 may be made of a material having a higher rigidity than the outer tube 330. For example, if the outer tube 330 is made of a polymer material, the coil tube 340 may be made of a metal material, for example stainless steel.

According to the embodiment in which the coil tube 340 having a spring shape is provided inside the operating tube 300, the rigidity of the operating tube 300 may be reinforced and the flexibility of the operating tube 300 may also be improved. For example, if the coil tube 340 is configured in a metal spring form, the rigidity of the operating tube 300 may be further enhanced by the metal material. In addition, in this case, since the coil tube 340 has a spring form, the operating tube 300 may be more smoothly inserted into or moved in the blood vessel. In particular, even if the coil tube 340 is made of a metal material, since the coil tube 340 has a spiral form, flexibility may be secured in a direction perpendicular to the longitudinal direction of the operating tube 300. Also, in this configuration, since the coil tube 340 may be formed using a single coil, the multiple-structured operating tube 300 may be manufactured more easily.

The coil tube 340 may be positioned inside the outer tube 330, so that at least the distal end of the coil tube 340 is not exposed to the outside of the operating tube 300. For example, the distal end of the coil tube 340 may be shorter than the distal end of the outer tube 330. In this case, the distal end of the coil tube 340 may be covered by the outer tube 330 and not exposed to the outside at the distal end of the operating tube 300.

According to this configuration of the present disclosure, since the distal end of the coil tube 340 is located inside the operating tube 300 and is not exposed to the outside, it is possible to prevent the distal end of the coil tube 340 from damaging other components of the catheter or the blood vessel. Also, in this configuration, even if the coil tube 340 is made of a metal material, it is possible to prevent the coil tube 340 from being corroded by blood or the like.

Meanwhile, in the configuration where the coil tube 340 is provided inside the outer tube 330 as described above, the inner tube 310 may be provided inside the coil tube 340.

Also preferably, the shaft body 600 may be configured to include an inner body, a mesh body, and an outer body. This will be described in more detail with reference to FIG. 9.

FIG. 9 is a perspective view schematically showing the shaft body 600 having a hollow in which the operating tube 300 is inserted according to an embodiment of the present disclosure. In FIG. 9, distal ends of the mesh body and the outer body are partially sectioned to show each component therein.

Referring to FIG. 9, the shaft body 600 may be configured in a triple structure having three layers of an inner body 610, a mesh body 620, and an outer body 630.

Here, the inner body 610 constitutes an inner layer of the shaft body 600, and a hollow may be formed in the longitudinal direction. In other words, the inner body 610 may elongate in one direction, and the fourth hollow may be formed.

In addition, the mesh body 620 may be configured in a mesh form to surround the outer surface of the inner body 610. For example, the mesh body 620 may be provided on the outer surface of the inner body 610 to elongate from the distal end to the proximal end of the inner body 610.

Moreover, the outer body 630 may be configured to elongate in one direction at the outside of the inner body 610 and the mesh body 620. In particular, the outer body 630 may be configured to surround the outer surfaces of the inner body 610 and the mesh body 620.

For the triple structure of the shaft body 600, the features of the operating tube 300 related to FIGS. 6 and 7 may be applied similarly, and these similar features will not described in detail. For example, the inner body 610 and the outer body 630 may be made of a polymer material. In addition, the mesh body 620 may be made of a metal material such as stainless steel to secure the rigidity of the operating tube 300. In particular, the mesh body 620 may be constructed in a woven mesh form using a plurality of woven wires. In addition, the mesh body 620 may be configured to have a plurality of unit mesh rings arranged successively along the longitudinal direction of the shaft body 600, similar to the operating tube 300 of FIG. 7.

At the distal end of the shaft body 600 having the triple structure, the mesh body 620 may be configured to be shorter than the inner body 610. In other words, the inner body 610 may be configured to protrude out of the distal end in comparison to the mesh body 620. For example, regarding the configuration of the distal end of the shaft body 600, the shaft body 600 may be configured such that the mesh body 620 is not provided up to about 10 cm from a terminal which is the outermost side of the distal end.

Since the mesh body 620 may be made of a material or structure that is more rigid than the inner body 610, if this configuration of the present disclosure is applied, the flexibility of the distal end of the shaft body 600 may be improved. Thus, the distal end of the shaft body 600 may be easily bent according to a curved shape of the blood vessel.

At this time, the shaft body 600 may be configured to include a coil body (not shown) instead of the mesh body 620. In other words, the shaft body 600 may include a coil body and an outer body 630, the outer body 630 may be configured in a form identical or similar to the former embodiment, and only the coil body may be configured in a different form from the mesh body 620.

Here, the coil body may be approximately configured in a form similar to the coil tube 340 of the operating tube 300 described in the embodiment of FIG. 8. In other words, the coil body may be configured in a coil form to surround the inner surface of the outer body 630.

With this configuration of the present disclosure, the shaft body 600 may have further improved rigidity and flexibility and be manufactured easier.

Meanwhile, the catheter according to the present disclosure may further include a power supply wire.

The power supply wire may be electrically connected to the electrode 500 to provide a power supply path to the electrode 500. For example, the power supply wire may allow an energy supply unit (not shown) to supply power to the electrode 500 by connecting its distal end to the electrode 500 and connecting its proximal end to the energy supply unit.

The power supply wire may be provided separately for each of a plurality of electrodes 500 and be electrically connected to each of the plurality of electrodes 500. At this time, the plurality of power supply wires may provide a power supply path for each of the plurality of electrodes 500. Here, the plurality of power supply wires may be configured to be separated from each other in a region from the electrodes 500 to the energy supply unit. Alternatively, the plurality of power supply wires may be configured so that one power supply wire is branched into two or more power supply wires at a predetermined point and each branched power supply wire is connected to each electrode 500.

The shaft body 600 may be configured in various ways so that the power supply wire is located therein.

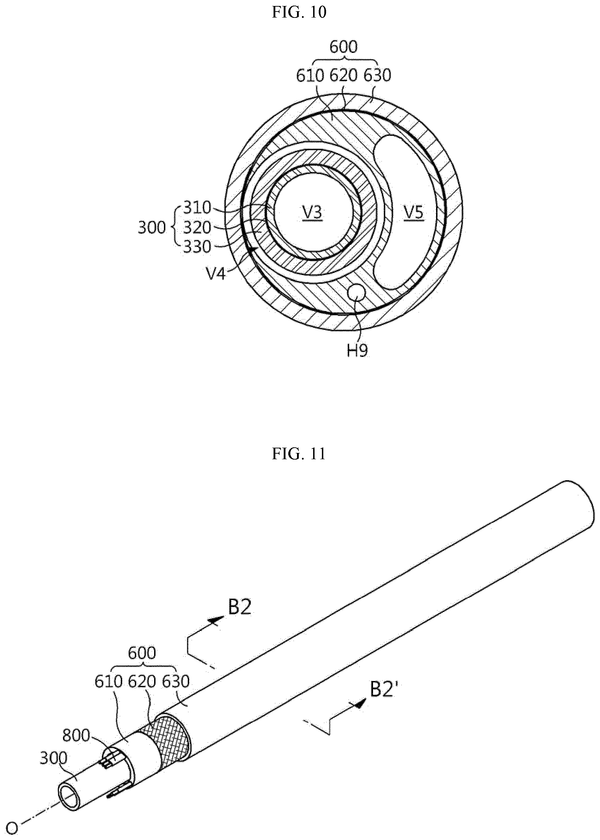

FIG. 10 is a cross-sectioned view, taken along the line B1-B1' of FIG. 9.

Referring to FIGS. 9 and 10, the shaft body 600 may have a fourth hollow as indicated by V4, and the operating tube 300 may be inserted into the fourth hollow. Further, in addition to the fourth hollow V4, the shaft body 600 may further include a hollow as indicated by V5. Also, this hollow is called a fifth hollow so as to be distinguished from other hollows explained above in this specification. In other words, in the shaft body 600, at least two hollows may be formed in the longitudinal direction.

The power supply wire may be inserted into the fifth hollow V5. In other words, the power supply wire may be inserted into the fifth hollow. Also, the power supply wire may be configured to be movable in the longitudinal direction inside the fifth hollow. In addition, the power supply wire may be extended from the proximal end of the catheter to the electrode 500 through the fifth hollow. At this time, the distal end and the proximal end of the fifth hollow may be configured to be opened to the outside so that the power supply wire is exposed.

According to this configuration of the present disclosure, both configurations for driving and electrically operating the catheter head may be provided within a very small outer diameter of the catheter. For example, the catheter may have a very small size of about 1.4 mm. In this configuration of the present disclosure, a configuration for opening/closing the catheter head inside the catheter of a small size and a configuration for inserting wires for electrical operation may be implemented more easily. In particular, in the embodiment having the fifth hollow, a space in which wires are inserted and moved may be easily secured. In addition, the configuration for driving the catheter head and the configuration for electrical operation may be mostly not exposed out of the catheter.

Further, the fifth hollow may be provided at an outer side of the fourth hollow. In other words, the fifth hollow may be biased to any side based on the central axis of the catheter. At this time, the fifth hollow may have a bent form along the outer circumference of the fourth hollow.

Namely, as shown in FIG. 10, the fourth hollow is formed substantially in a circular shape, but the fifth hollow may not be formed in a circular shape. More specifically, the fifth hollow may be interposed between the surface of the fourth hollow and the outer surface of the shaft body 600, and may be formed in a bent shape along a portion of the rim of the fourth hollow and a portion of the outer rim of the shaft body 600.

According to this configuration of the present disclosure, the fifth hollow may have a large size without relatively increasing the outer diameter of the shaft body 600. Thus, a space in which the power supply wire is inserted may be secured widely. Therefore, a process of inserting the power supply wire into the shaft body 600 may be easily performed. In particular, since the catheter may include a plurality of electrodes 500, a plurality of power supply wires should be provided to supply power the plurality of electrodes 500. Therefore, if the wide fifth hollow has a large area as in this embodiment, it is easy to provide a configuration in which power is supplied to a plurality of electrodes 500.

Moreover, the shaft body 600 may include a variety of wires other than the power supply wire. For example, the catheter head may include one or more sensors, including a temperature sensor, and at this time, a wire for sensing needs to extend long from the proximal end to the distal end of the catheter. In this case, wires for sensing other than the power supply wire may be inserted through the broad space of the fifth hollow.

Meanwhile, in the embodiment in which the shaft body 600 has the triple structure of the inner body 610, the mesh body 620 and the outer body 630, the fourth hollow and the fifth hollow may be formed at the inner body 610 as shown in FIG. 10.

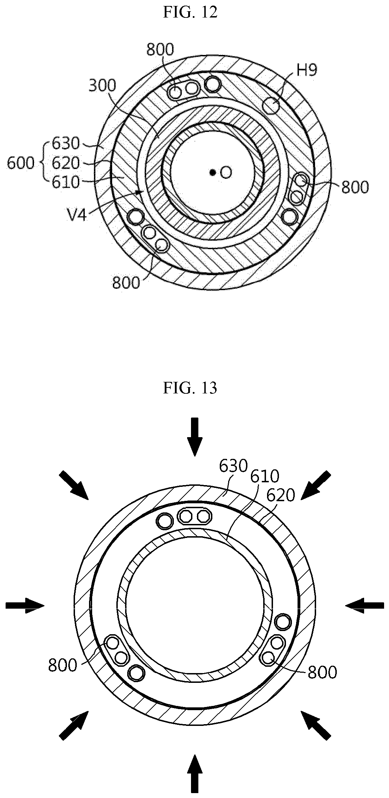

FIG. 11 is a perspective view schematically showing a catheter according to another embodiment of the present disclosure, and FIG. 12 is a cross-sectioned view, taken along the line B2-B2' FIG. 11. For the configuration depicted in FIGS. 11 and 12, the features which are similar to the above description of the former embodiments are not described in detail, and only different features will be mainly described.

Referring to FIGS. 11 and 12, the shaft body 600 may be configured so that the central axis of the fourth hollow coincides with the central axis of the catheter. In other words, the fourth hollow of the shaft body 600 may be located in the middle of the catheter. At this time, both the central axis of the fourth hollow and the central axis of the catheter are designated by O in the figures and may be regarded as being identical to each other.

In this configuration, if the catheter includes a plurality of power supply wires 800, the plurality of power supply wires 800 may be disposed to be spaced apart from each other by a predetermined angle on the basis of the central axis of the fourth hollow. For example, as shown in FIGS. 11 and 12, if the catheter includes three power supply wires 800, the power supply wires 800 may be disposed at intervals of 120 degrees on the basis of the central axis O of the catheter.

According to this configuration of the present disclosure, the shape of the section of the catheter perpendicular to the longitudinal direction may be symmetrical on the basis of the center point O in the up, down, right and left directions. Thus, the catheter head symmetrically arranged in the up, down, right and left directions on the basis of the center point of the catheter may be easily connected to the operating tube 300 located in the fourth hollow.

Moreover, in this case, the catheter head may be opened and closed stably. In other words, the catheter head may be opened and closed radially with respect to the central axis of the catheter. Here, if the operating tube 300 is located at the center of the catheter, this radial opening/closing operation may be stably implemented.

In addition, the power supply wire 800 may be inserted between the inner surface and the outer surface of the shaft body 600 and fixedly coupled to the shaft body 600. At this time, the inner surface of the shaft body 600 may be regarded as a surface which forms the fourth hollow of the shaft body 600.

In other words, the power supply wire 800 is not configured to be inserted into a hole formed in the shaft body 600, but the power supply wire 800 may be configured to be embedded in the shaft body 600, namely to be integrated thereto, as shown in FIG. 12.

According to this configuration of the present disclosure, since the power supply wire 800 is already included in the shaft body 600 during the manufacturing process, there is no need to perform a process of separately inserting the power supply wire 800 in order to assemble the catheter. Moreover, since it is not needed to prepare a hole for inserting the power supply wire 800 in the shaft body 600, the outer diameter of the shaft body 600 is reduced, which is advantageous for miniaturization of the catheter. In addition, since the power supply wire 800 is fixed inside the shaft body 600, there is no fear that the power supply wire 800 is damaged due to the fluctuation of the power supply wire 800 or its portion connected to the electrode 500 is broken.

In particular, when the shaft body 600 has a triple structure of the inner body 610, the mesh body 620 and the outer body 630, the power supply wire 800 may be embedded in the inner body 610.

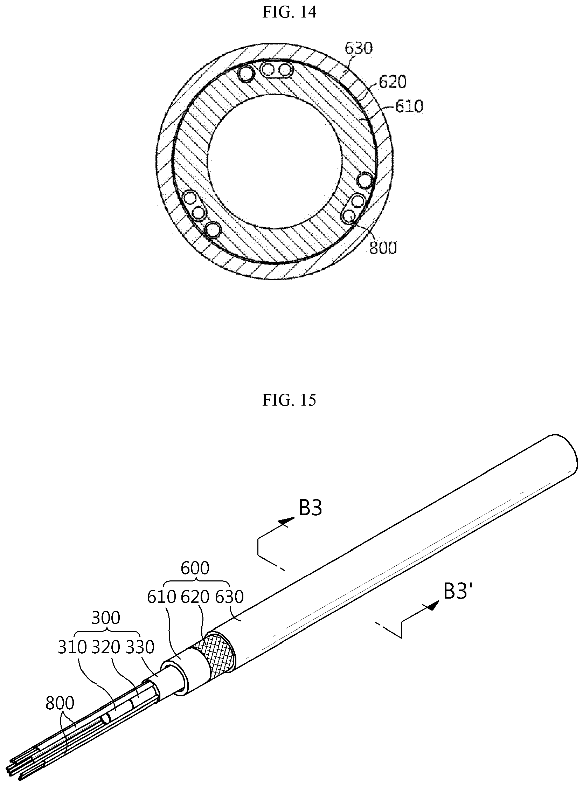

FIGS. 13 and 14 are schematic views showing that the power supply wire 800 is included in the shaft body 600 according to an embodiment of the present disclosure.

First, referring to FIG. 13, the power supply wire 800 may be interposed between the inner body 610 and the mesh body 620. The outer body 630 may be located outside the mesh body 620. At this time, the inner body 610 and the outer body 630 are made of a polymer material, and the mesh body 620 may be made of a metal material. In this configuration, as indicated by an arrow, if heat and high pressure are applied from the outside of the outer body 630, the outer body 630 may be partially melted.

In addition, the molten outer body 630 partially flows inwards through the mesh-shaped opening of the mesh body 620, and as shown in FIG. 14, the outer body 630 may be coupled to the existing inner body 610 while covering the power supply wire 800. In addition, if the applied heat and pressure are released, a part of the outer body 630 melted and flowing into the mesh body 620 is cooled to form an inner body 610 together with the existing inner body 610.

Meanwhile, even though it has been illustrated in FIGS. 13 and 14 that the mesh body 620 is included, the mesh body 620 may not be included. In addition, the inner body 610 and the outer body 630 may be made of the same material.

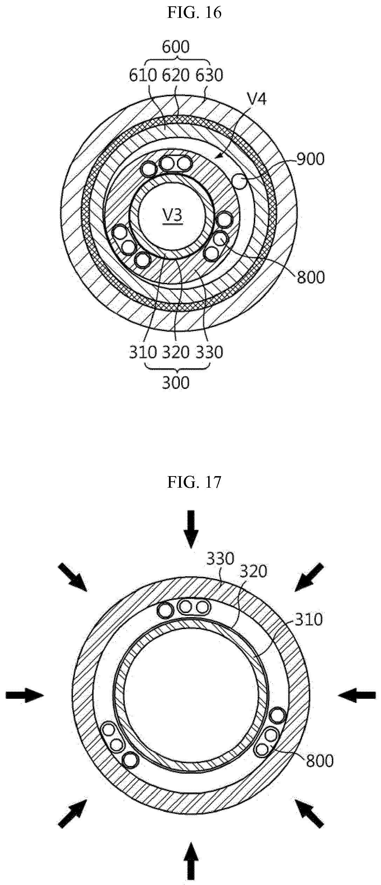

FIG. 15 is a perspective view schematically showing a catheter according to another embodiment of the present disclosure, and FIG. 16 is a cross-sectioned view, taken along the line B3-B3' of FIG. 15. In the configuration of FIGS. 15 and 16, the features which are similar to the above description of the former embodiments are not described in detail, and only different features will be mainly described.

Referring to FIGS. 15 and 16, similar to the embodiment of FIGS. 11 and 12, the fourth hollow of the shaft body 600 is located at the center of the catheter, and a plurality of power supply wires 800 are disposed to be spaced by a predetermined angle from the central axis of the fourth hollow. Thus, similar to FIGS. 11 and 12, the catheter head may be easily connected to the operating tube 300 and the power supply wire 800, and the catheter head may be stably operated by means of the operating tube 300.

However, different from FIGS. 11 and 12, in this embodiment, the power supply wire 800 is embedded in the operating tube 300. In other words, the power supply wire 800 is inserted between the inner surface and the outer surface of the operating tube 300, and is integrated with and fixedly coupled to the operating tube 300.

According to this configuration of the present disclosure, since the power supply wire 800 is already included in the operating tube 300 during the manufacturing process, it is not needed to perform a process of inserting the power supply wire 800 into the shaft body 600 or the operating tube 300 for assembling the catheter. In addition, it is not needed to prepare a hole for inserting the power supply wire 800 in the shaft body 600 or the operating tube 300, the catheter head may be easily designed in a small size, and it is possible prevent that the power supply wire 800 is damaged or its portion connected to the electrode 500 is broken.

In particular, if the operating tube 300 has a triple structure of the inner tube 310, the mesh tube 320 and the outer tube 330, the power supply wire 800 may be embedded in the outer tube 330, as shown in the figures.

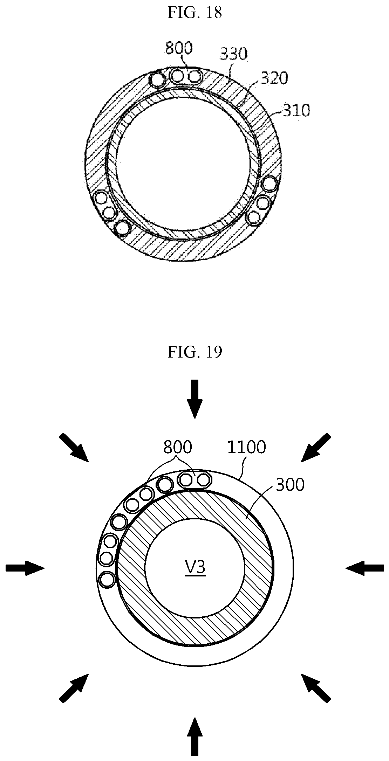

FIGS. 17 and 18 are schematic views showing that the power supply wire 800 is included in the operating tube 300 according to an embodiment of the present disclosure.

First, referring to FIG. 17, the power supply wire 800 may be interposed between the mesh tube 320 and the outer tube 330. In addition, the inner tube 310 may be positioned inside the mesh tube 320. At this time, the outer tube 330 and the inner tube 310 are made of a polymer material, and the mesh tube 320 may be made of a metal material. In this configuration, as indicated by an arrow, if heat and high pressure is applied from the outside of the outer tube 330, the outer tube 330 may be melted at least partially.

In addition, a part of the molten outer tube 330 may be directed toward the mesh tube 320 while covering the power supply wire 800, as shown in FIG. 18. Moreover, the melt of the outer tube 330 reaching the mesh tube 320 may pass through the mesh-shaped opening of the mesh tube 320 and come into contact with the inner tube 310. Thus, the melt of the outer tube 330 may become a part of the inner tube 310 while being coupled to the inner tube 310, and the power supply wire 800 may be embedded in the outer tube 330.

Meanwhile, even in the embodiment where the power supply wire 800 is embedded in the operating tube 300, similar to the configuration of FIG. 14, the power supply wire 800 may be located inside the mesh tube 320 and be embedded in the inner tube 310. In addition, in the configuration of FIG. 8, the power supply wire 800 may be located inside the operating tube 300. In particular, the power supply wire 800 may be configured to be embedded inside the outer tube 330.

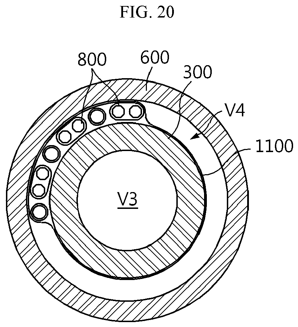

In addition, the power supply wire 800 may be provided in contact with the outer surface of the operating tube. In this case, the catheter according to the present disclosure may further include a thermally shrinkable film that makes the power supply wire 800 to closely adhere to the outer surface of the operating tube 300. This will be described in more detail with reference to FIGS. 19 and 20.

FIG. 19 is a schematic view showing that the power supply wire 800 is provided at an outer side of the operating tube 300 according to another embodiment of the present disclosure, and FIG. 20 is a schematic view showing a catheter to which the configuration of FIG. 19 is applied. This embodiment will also be explained based on features different from the former embodiments.

First, referring to FIG. 19, the power supply wire 800 may be configured to be located at the outside of the operating tube 300. In addition, a thermally shrinkable film 1100 having a tube form may be configured to surround the power supply wire 800 and the operating tube 300 together. In other words, the thermally shrinkable film 1100 has a tube shape elongating in the same direction as the longitudinal direction of the catheter, and the power supply wire 800 and the operating tube 300 may be inserted into an inside (a hollow) of the thermally shrinkable film 1100. In addition, as indicated by an arrow, if heat is applied from the outside of the thermally shrinkable film 1100, the thermally shrinkable film 1100 may be shrunken due to the heat and be adhered to the outer sides of the power supply wire 800 and the operating tube 300.

If so, the thermally shrinkable film 1100 may be configured to surround the outer sides of the power supply wire 800 and the operating tube 300 in a thermally shrunken state, as shown in FIG. 20. Thus, the power supply wire 800 may be attached to the outer side of the operating tube 300 by means of the thermally shrinkable film 1100. In addition, the thermally shrinkable film 1100, the power supply wire 800 and the operating tube 300 configured as above may be located in an inner space of the shaft body 600, namely in the fourth hollow V4.

Here, the thermally shrinkable film 1100 may be made of a material which may be shrunken by heat. For example, the thermally shrinkable film 1100 may be made of a polymer material, for example PET (Poly Ethylene Terephthalate). This material facilitates the thermal shrinking process, does substantially not generate harmful substances during the thermal shrinking process, and ensures excellent flexibility.

According to this configuration of the present disclosure, since the power supply wire 800 is fixed to the outer side of the operating tube 300, the risk of disconnection is low, and it is possible to prevent that the movement of the operating tube 300 is restricted by the power supply wire 800 or the operating tube 300 and the shaft body 600 are damaged. In addition, the process of fixing the power supply wire 800 to the operating tube 300 and the process of inserting the power supply wire 800 into the shaft body may be more easily performed, thereby improving the manufacturing processability of the catheter. In addition, even if the catheter does not have a large diameter, a structure for opening/closing and electrically operating the catheter may be provided in the catheter. Also, in this case, the head portion of the catheter may be shortened, the catheter may become flexible, and the exposure of the power supply wire 800 may be limited to ensure easy protection of the power supply wire 800. Moreover, according to this configuration, the operating tube 300 is positioned at the center of the catheter, and thus the guide wire 700 may be easily inserted therein.

Also preferably, the catheter according to the present disclosure may further include a deflection wire 900.

The deflection wire 900 elongates in one direction and may be located from the distal end of the catheter to the proximal end of the catheter. At this time, the distal end of the deflection wire 900 may be fixed to the distal end of the catheter. Also, the proximal end of the deflection wire 900 may be exposed outwards at the proximal end of the shaft body 600.

In addition, the deflection wire 900 may be configured to be movable in the longitudinal direction in the inner space of the catheter. In particular, the deflection wire 900 may be configured to be movable in the longitudinal direction in a state of being inserted into the shaft body 600.

For example, as shown in FIG. 16, the deflection wire 900 may be interposed between the inner surface of the operating tube 300 and the inner surface of the shaft body 600, in the inner space of the fourth hollow V4 which is a hollow of the shaft body 600. Alternatively, in the configuration of FIG. 10 or FIG. 12, a hole may be formed long in the inner body 610 of the shaft body 600 along the longitudinal direction of the catheter as indicated by H9, and the deflection wire 900 may be inserted into the hole to be moved in the longitudinal direction.

According to this configuration of the present disclosure, the catheter head at which the distal end of the shaft body 600 is located may be bent by means of the deflection wire 900. For example, if the deflection wire 900 is pulled by a surgical operator, the distal end of the shaft body 600 may be bent, and if the deflection wire 900 is pushed by the surgical operator, the distal end of the shaft body 600 may be spread. Accordingly, the distal end of the shaft body 600 may be bent according to the curved shape of a blood vessel by means of the deflection wire 900, which allows the catheter head to be inserted smoothly along the blood vessel and prevents the inside of the blood vessel from being damaged.

In addition, the catheter according to the present disclosure may further include an end tip 1000 at a front side of the distal end of the catheter, namely the distal end of the catheter head, as shown in FIG. 1 or the like.

The end tip 1000 may have a tube shape with a hollow and be made of soft and flexible material. In particular, the end tip 1000 may be made of a composition containing polyether block amide (PEBA).

In this configuration of the present disclosure, when the end of the catheter moves along a blood vessel or the like, the end tip 1000 made of soft and flexible material is located at a foremost position, which may reduce damages to the blood vessel and facilitate easier change of a moving direction. Further, the end tip 1000 made of the above material may be photographed by X-ray, and thus a location of the catheter head may be easily figured out.

A denervation apparatus according to the present disclosure may include the catheter according to the present disclosure. In addition, the denervation apparatus may further include an energy supplying unit and an opponent electrode 500 in addition to the catheter for denervation. Here, the energy supplying unit may be electrically connected to the electrode 500 through the power supply line 800. In addition, the opponent electrode 500 may be electrically connected to the energy supplying unit through another wire. In this case, the energy supplying unit may supply energy to the electrode 500 of the catheter in the form of high frequency or the like, and the electrode 500 of the catheter generates heat to ablate nerves around the blood vessel, thereby blocking the nerves.

The present disclosure has been described in detail. However, it should be understood that the detailed description and specific examples, while indicating preferred embodiments of the disclosure, are given by way of illustration only, since various changes and modifications within the spirit and scope of the disclosure will become apparent to those skilled in the art from this detailed description.

In addition, even though terms representing directions such as proximal, distal, upper, lower, right, left or the like have been used in the specification, the terms are just used to indicate relative locations for convenience and can be replaced with other words according to an observation point of an observer or an arrangement of a component, as obvious to those having ordinary skill in the art.

### Reference Signs

100: first support member
200: second support member
300: operating tube
310: inner tube, 320: mesh tube, 330: outer tube, 340: coil tube
400: connection member
500: electrode
600: shaft body
610: inner body, 620: mesh body, 630: outer body
700: guide wire
800: power supply wire
900: deflection wire
1000: end tip
1100: thermally shrinkable film

## Claims

1. A catheter, which elongates in one direction and has a proximal end and a distal end, the catheter comprising:
a first support member located near the distal end of the catheter and having a first hollow with both open ends;
a second support member located near the proximal end of the catheter in comparison to the first support member and having a second hollow with both open ends;
an operating tube configured to elongate in one direction and having a third hollow with both open longitudinal ends, the operating tube being inserted into the first hollow and the second hollow and fixed to the first support member or the second support member to move the first support member or the second support member in a longitudinal direction;
at least one connection member having one end connected to the first support member and the other end connected to the second support member, wherein when a distance between the first support member and the second support member decreases, at least a part of the connection member is bent so that the bending portion becomes away from a central axis of the third hollow;
at least one electrode provided at the connection member to generate heat; and
a shaft body located near the proximal end of the catheter in comparison to the second support member to elongate in one direction, the shaft body having a fourth hollow with both open longitudinal ends so that the operating tube is inserted therein and movable in the longitudinal direction.

2. The catheter according to claim 1, further comprising:
a guide wire configured to elongate in one direction and inserted into the third hollow through both open ends of the operating tube to be movable in the longitudinal direction inside the third hollow.

3. The catheter according to claim 1,
wherein the operating tube is fixed to the first support member and is movable in the longitudinal direction inside the second hollow.

4. The catheter according to claim 1, wherein the operating tube includes:
an inner tube configured to elongate in one direction and having the third hollow;
a mesh tube having a mesh form and configured to surround an outer surface of the inner tube; and
an outer tube configured to elongate in one direction and surround the outer surfaces of the inner tube and the mesh tube.

5. The catheter according to claim 1, wherein the operating tube includes:
an outer tube configured to elongate in one direction and having the third hollow; and
a coil tube having a coil form and configured to surround an inner surface of the outer tube.

6. The catheter according to claim 1, wherein the shaft body includes:
an inner body configured to elongate in one direction and having the fourth hollow;
a mesh body having a mesh form and configured to surround an outer surface of the inner body; and
an outer body configured to elongate in one direction and surround the outer surfaces of the inner body and the mesh body.

7. The catheter according to claim 1, further comprising:
a power supply wire electrically connected to the electrode to give a power supply path to the electrode.

8. The catheter according to claim 7,
wherein the shaft body further has a fifth hollow formed therein in the longitudinal direction, separately from the fourth hollow, and
wherein the power supply wire is configured to be inserted into the fifth hollow and movable in the longitudinal direction, and is connected from the proximal end of the catheter to the electrode.

9. The catheter according to claim 8,
wherein the fifth hollow is provided at one outer side of the fourth hollow and is bent along an outer circumference of the fourth hollow.

10. The catheter according to claim 7,
wherein the shaft body is configured so that a central axis of the fourth hollow coincides with a central axis of the catheter, and
wherein the power supply wire is provided in plural so that the power supply wires are arranged to be spaced apart from each other by a predetermined angle on the basis of the central axis of the fourth hollow.

11. The catheter according to claim 10,
wherein the power supply wires are inserted between an inner surface and an outer surface of the shaft body and are fixedly coupled to the shaft body.

12. The catheter according to claim 10,
wherein the power supply wires are inserted between an inner surface and an outer surface of the operating tube and are fixedly coupled to the operating tube.

13. The catheter according to claim 7,
wherein the power supply wire is provided in contact with an outer surface of the operating tube, and
wherein the catheter further comprises a thermally shrinkable film having a thermally shrunken form to surround outer sides of the power supply wire and the operating tube.

14. The catheter according to claim 1, further comprising:
a deflection wire configured to elongate in one direction and having a distal end fixed to a distal end of the shaft body and a proximal end exposed outwards at a proximal end of the shaft body, the deflection wire being configured to be movable in the longitudinal direction inside the shaft body.

15. A denervation apparatus, comprising the catheter defined in any one of claims 1 to 14.
